# EUROPEAN PATENT SPECIFICATION

(11) **EP 2 621 886 B1**
(45) Date of publication and mention of the grant of the patent: **02.05.2018**
(21) Application number: 11758448.2
(22) Date of filing: 15.09.2011
(51) Int. Cl.: C07C 205/16, C07C 209/34, C07C 209/70, C07C 215/28

(54) **PROCESS FOR MAKING FINGOLIMOD**
VERFAHREN ZUR HERSTELLUNG VON FINGOLIMOD
PROCÉDÉ DE FABRICATION DE FINGOLIMOD

(30) Priority: 22.12.2010 WO PCT/EP2010/070535; 01.10.2010 WO PCT/EP2010/006039
(43) Date of publication of application: 07.08.2013
(73) Proprietor: Synthon BV, 6545 CM Nijmegen (NL)
(72) Inventor: GIELING, Reinerus Gerardus, 6545 CM Nijmegen (NL)
(74) Representative: Mendivil Gil, Maria Dolores
(86) International application number: PCT/EP2011/065975
(87) International publication number: WO 2012/041707

(56) References cited:
- CN-A- 1 765 872
- BISWAJIT KALITA, NABIN C. BARUA, MAITREYEE BEZBARUA, GHANASHYAM BEZ: "Synthesis of 2-Nitroalcohols by Regioselective Ring Opening of Epoxides with MgSO4/MeOH/NaNO2 System: A Short Synthesis of Immunosuppressive Agent FTY-720", SYNLETT, 2001, pages 1411-1414, XP002607731, DOI: 10.1055/s-2001-16776 cited in the application

## Description

### BACKGROUND OF THE INVENTION

Fingolimod (often coded as FTY 720), chemically 2-amino-2-[2-(4-octylphenyl)ethyl]-propane-1,3-diol of the formula (1) 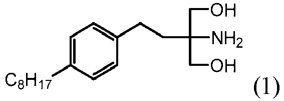 is a pharmaceutically active compound currently tested as an immunosuppressive drug and as an active agent in treatment of multiple sclerosis. It may form stable acid addition salts, of which fingolimod hydrochloride is the most common one.

Fingolimod has been first disclosed in EP 627406 of Yoshitomi, where also two basic routes for making it have been described.

In the first route (Example 28 of EP'406), the last synthetic step comprises deacetylation of protected diol-amine (2) by a basic hydrolysis

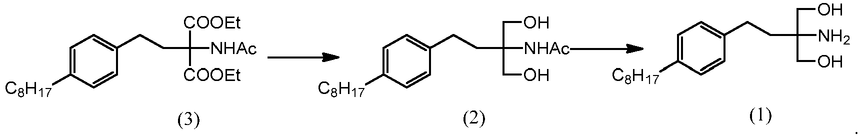

In the second route (Example 234of EP'406), the last synthetic step comprises reduction of a diester-amine (4)

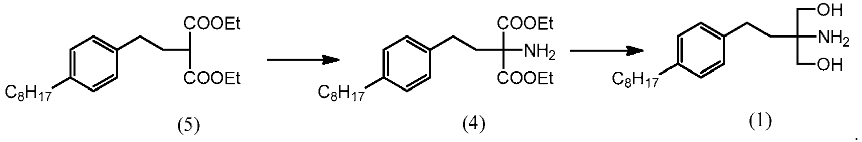

Subsequently, several alternate routes for making fingolimod were developed. Among them, processes employing reduction of a suitable nitro-intermediate are of considerable interest. The first such process, disclosed by Kalita et al. in Synlett 2001, No.9, 1411-1414, comprises, in the last step, a reduction of a nitro-diol (6), which has been prepared by a reaction of a nitro-alkane (7) with paraformaldehyde:

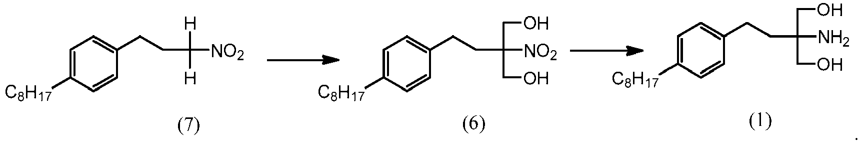

Chinese patent CN 1310869C has disclosed a second process, in which fingolimod is made by a reduction/hydrogenolysis of a hydroxylated nitrodiol (8). The conversion of (8) to fingolimod may be either direct, or may run via the above nitrodiol (6):

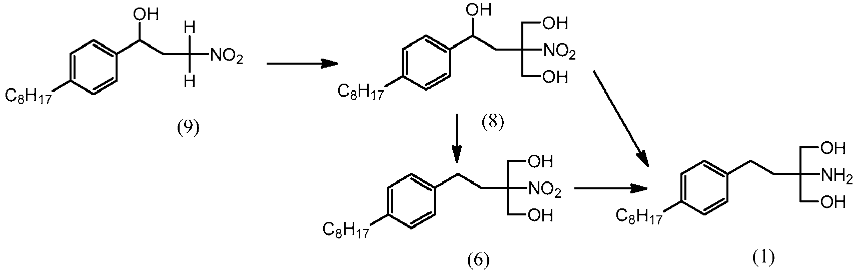

A third process was disclosed in Chinese patent CN 1212308C and comprises reduction of a nitro-diester (10):

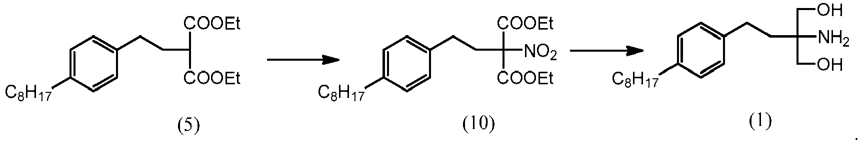

When comparing these known routes, it appears that the most suitable starting material is the hydroxylated nitroalkane (9) as it may be made from available raw materials by the shortest and most economic way. However, the sequential or parallel reduction/hydrogenolysis of both the OH- and the NO₂-groups of the intermediate (8) is very slow and thus less economical.

Thus, while many processes are known for making fingolimod, an improvement in the matter is still desirable.

### BRIEF DESCRIPTION OF THE PRESENT INVENTION

In a first aspect, the present invention provides for a new compound of formula (11) and/or (14) 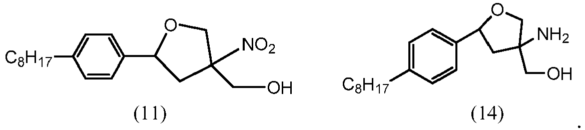 In a second aspect, the invention provides for a process for making the compound of formula (11) comprising reacting the compound of formula (8) 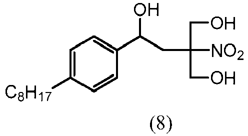 with a strong acid, most preferably p-toluene sulfonic acid, in an organic anhydrous solvent.

In a third aspect, the invention provides for a process of making fingolimod of formula (1), 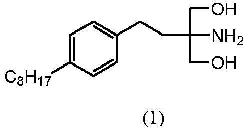 or an acid addition salt thereof,
comprising a step of reacting the compound of formula (11) with hydrogen in the presence of a hydrogenation catalyst, preferably palladium catalyst, optionally followed by converting fingolimod of formula (1) into an acid addition salt.

In a specific aspect, the present invention also provides for a process of making fingolimod of formula (1), or an acid addition salt thereof, comprising the step of reacting the compound of formula (8) in a non-aqueous solvent with hydrogen under catalysis of a hydrogenation catalyst, preferably palladium catalyst, and in presence of a strong acid, preferably p-toluene sulfonic acid or hydrogen chloride.

In a further aspect, the invention provides a process for making the compound (8) comprising the reaction of compound (9) 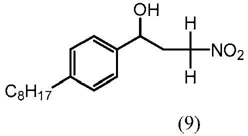 with a solution of formaldehyde in a mixture of water and methanol.

The use of compound (11) in making fingolimod forms another specific aspect of the present invention.

### BRIEF DESCRIPTION OF THE DRAWINGS

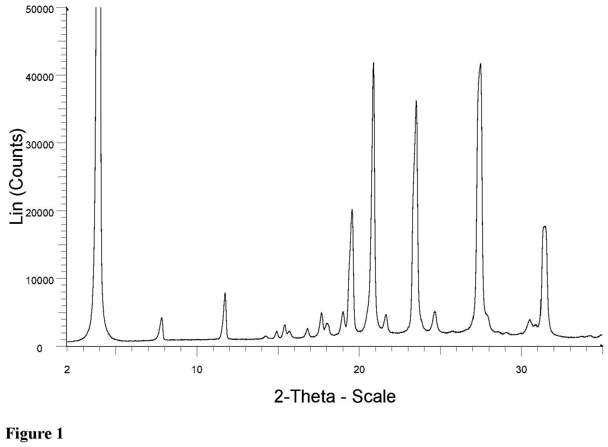

Figure 1. XRPD pattern of fingolimod prepared by the process of Example 10.

### DETAILED DESCRIPTION OF THE INVENTION

The present invention deals with an improved process for making the compound fingolimod of formula (1), or an acid addition salt thereof, from the compound of formula (9), which exhibits various advantages over other ways of conversion of compound (9) to the compound (1) known in the art. The advantages are discussed on the relevant places of further description.

The "acid addition salts" as used throughout the invention are typically those allowed for pharmaceutical use by regulatory authorities, e.g., hydrochloride, hydrobromide, sulphate, nitrate, phosphate, formate, acetate, propionate, oxalate, malonate, maleate, fumarate, citrate, malate and the like. These acid addition salts may be obtained by any conventional methods

In essence, the new process, which the present invention deals with, may be depicted by the following scheme:

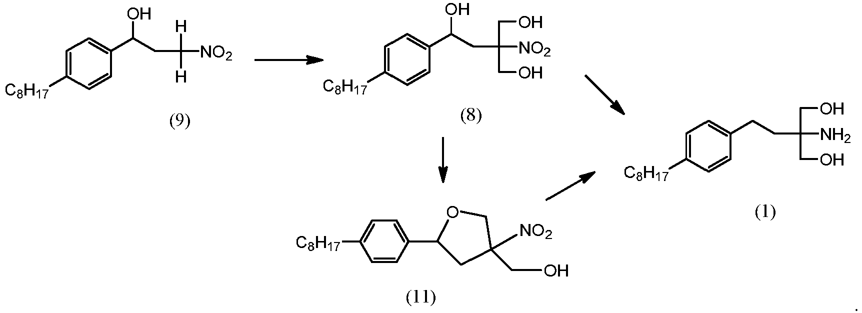

The starting material of the process, the nitro-alcohol compound of formula (9), is a known compound. The process of making it has been disclosed in CN 1310869 and is based on a reduction of a nitro-ketone of formula (12) with sodium borohydride. While this process is generally useful, it suffers from a problem, that it is sometimes accompanied with a formation of a des-nitro impurity of formula (13).

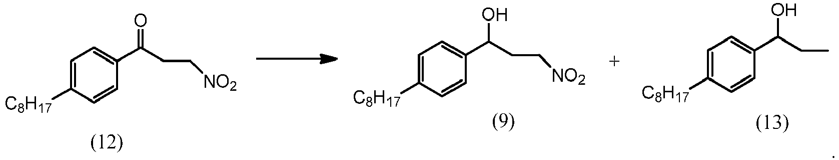

It was found by the present inventors that replacing the sodium borohydride by lithium borohydride decreases the amount of the impurity (13) in the reaction product without requiring longer reaction times or inconvenient reaction temperatures. Thus, advantageously, the compound (9) may be made by reacting the nitroketone of formula (12) by lithium borohydride in a solvent. The useful reaction solvent is, e.g., tetrahydrofuran. The convenient reaction temperature is from -20 to 0°C. The molar ratio of the starting ketone and the hydride is advantageously about 2 : 1. The course of the reaction may be advantageously monitored by a suitable analytical technique, e.g., by HPLC or TLC. After the reaction is complete, the reaction product may be isolated from the reaction mixture, e.g. by extraction from an aqueous solution with a suitable water immiscible solvent.

The nitro-alcohol (9) is converted to the next fingolimod intermediate, a hydroxylated nitro-diol (8), by a hydroxymethylation reaction with two molecules of formaldehyde.

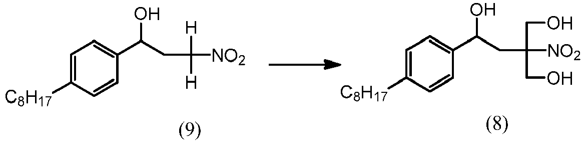

The prior art (CN '869) uses paraformaldehyde as the source of formaldehyde. The process employing paraformaldehyde however suffers from a certain disadvantage, as a formaldehyde polymer is formed in the reaction mixture. This formaldehyde polymer very firmly adheres on walls of reaction vessels and auxiliary equipment such as stirrers and thermometers, which requires extensive cleaning of the vessel and auxiliaries after the reaction. The polymer also impurifies the reaction mixture and the reaction product. It was found by the present inventors that paraformaldehyde may be advantageously replaced by an aqueous solution of formaldehyde stabilized by methanol (formalin). The reaction then may be performed in an aqueous environment, which is economically advantageous, whereby the methanol present in the formalin agent stabilizes formaldehyde against forming undesirable polymers.

Thus, in a suitable arrangement of reaction conditions, the nitro-alcohol (9) reacts with an aqueous solution of formaldehyde under presence of methanol, which advantageously is the commercially available 20 % or 37% solution of formaldehyde in water comprising about 10% of methanol.

The reaction temperature is advantageously from 30 to 60°C, preferably from 45 to 50°C. Useful molar ratio between compound (9) and formaldehyde is from 1 : 3 to 1 : 8. The course of the reaction may be advantageously monitored by a suitable analytical technique, e.g. by HPLC or TLC. After the reaction is complete, the reaction product may be isolated from the reaction mixture, e.g. by an extraction from an aqueous solution by a water-immiscible solvent.

In an alternate mode, the hydroxymethylation of (9) may be also performed by a reaction with methylal (formaldehyde dimethylacetal).

In subsequent steps, the hydroxylated nitro-diol (8) is converted to fingolimod (1) by substitution of the alpha-positioned to the benzene ring (in further "benzylic") OH-group by hydrogen and reduction of the nitro-group to amino group. According to CN'869, the conversion may be either direct, by a 48 hour hydrogenation by hydrogen catalyzed by Pd/C, performed in concentrated aqueous HCl and methanol, or it may run indirectly via the compound (6). The conversion to the compound (6) by Pd-catalyzed hydrogenation takes also 48 hours, and the subsequent reduction of the compound (6) to the desired product takes the next 20 hours. Thus, it is evident that the reaction times are extremely long, which makes the process economically very inconvenient.

Now it was found out that the overall conversion time of the compound (8) to fingolimod (1) may be dramatically decreased if the compound (8) is subjected to a reaction with a strong acid in an inert organic solvent, which is typically non-aqueous. Such reaction of the compound (8) with the strong acid may occur prior to or simultaneously with the hydrogenation reaction. The preferred strong acid is a sulfonic acid, preferably methane sulfonic acid, benzene sulfonic acid and most preferably p-toluene sulfonic acid, anhydrous hydrogen chloride or hydrogen bromide, sulphuric acid, phosphoric acid, perchloric acid, trifluoroacetic acid. Strongly acidic ion-exchange resins may also serve as a strong acid for purpose of this process. The preferred inert nonaqueous solvent is an aliphatic or aromatic hydrocarbon of 5 to 12 carbons, most preferably toluene, an aliphatic alcohol of 1 to 6 carbons, most preferably methanol and/or an aliphatic ester, most preferably ethyl acetate.

The reactive contact of the compound (8) with a strong acid in an inert nonaqueous solvent, results in removing the benzylic OH- group and in forming a substrate, which, as will appear in the next steps, is very reactive in the hydrogenation reaction. Due to this reactive contact of the compound (8) with the acid in the nonaqueous solvent, the hydrogenation to the compound (1) may be finalized in less than 4 hours.

Dependent on the amount of the strong acid and mutual timing of both reactions, the overall mechanism of the conversion of compound (8) to fingolimod (1) is apparently as follows:

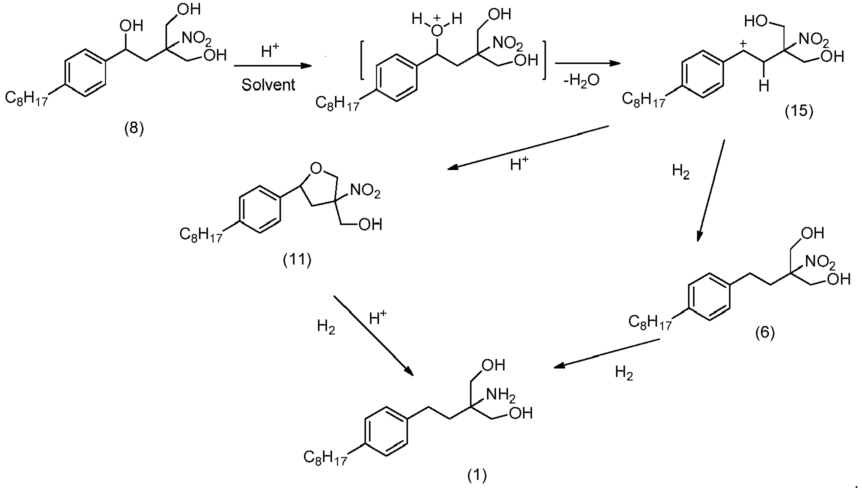

In the first step, the strong acid first protonates the benzylic -OH group. In a nonaqueous solvent, a molecule of water is then split upon forming a reactive substrate of the formula (15). Its further fate depends on the reaction conditions.

### Variant a]

If treated with sufficient amount of the strong acid for a sufficient time, the unstable compound (15) is stabilized by cyclization upon forming the compound of formula (11). Such compound is adequately reactive for the hydrogenation reaction; it can alternately be isolated from the reaction mixture, if desirable, and subjected to the hydrogenation reaction in a subsequent step.

Thus, in a non-limiting example of the process, the compound (8) is heated, preferably at a temperature of at least 40°C and most preferably under reflux conditions, with p-toluene sulfonic acid or other suitable strong acid in a suitable inert nonaqueous solvent, which is typically an aliphatic or aromatic hydrocarbon, preferably of 5 to 12 carbon atoms, most preferably toluene, or an aliphatic alcohol, preferably of 1 to 6 carbon atoms, most preferably methanol. Useful molar ratio between the compound of formula (8) and the strong acid is from 10 : 1 to 1 : 3. The course of the reaction may be advantageously monitored by a suitable analytical technique, e.g., by HPLC or TLC. After the reaction is complete, the reaction product may be isolated from the reaction mixture by an extraction of an alkalinized aqueous solution with a water-immiscible organic solvent, e.g. by toluene or ethyl acetate. However, it is possible, and in some aspects even advantageous, not to isolate the compound of formula (11) from the reaction mixture and to subject this reaction mixture to the subsequent hydrogenation reaction directly, in a so-called one-pot arrangement.

The compound of formula (11), either in its isolated form or in a reaction mixture obtained by the preceded step, is converted to the desired fingolimod of the formula (1) by a reaction with hydrogen under catalysis by a suitable hydrogenation catalyst, such as a palladium- or platinum comprising catalyst. Other suitable catalyst may be, e.g., Raney-nickel. If the compound of formula (11) has been isolated, then the hydrogenation reaction runs in a suitable inert solvent, e.g. in an aliphatic or aromatic hydrocarbon such as toluene or in an aliphatic alcohol such as methanol; otherwise the reaction mixture serves as the reaction medium. Under comparative reaction conditions to those disclosed in the prior art (CN '869), i.e. using Pd/C as the hydrogenation catalyst, the reaction time may take 60 - 180 minutes, i.e. is dramatically shorter.

The compound of formula (11) is thus a very useful intermediate for making fingolimod, as it provides the desired product by a far shorter process than that of the prior art.

In an advantageous arrangement, the compound of formula (11) is subjected to the hydrogenation reaction under catalysis by palladium on carbon. Yet advantageously, the hydrogenation is performed under a hydrogen pressure of about 30 - 50 bar and/or at a temperature from 25 to 100°C. The course of the reaction may be advantageously monitored by a suitable analytical technique, e.g. by HPLC or TLC. After the reaction is complete, the reaction product may be isolated from the reaction mixture, e.g. by an extraction of an alkalinized aqueous solution with a water-immiscible solvent, e.g. by toluene or ethyl acetate.

It should be noted that the compound of formula (11) has two chiral carbons and may exist in four (two pairs of) diastereomers differing by spatial orientation of substituents at the five-membered ring. Any of the diastereomers is equally suitable for making fingolimod according to the present invention. Therefore, the compound of formula (11) may be used in the process for making fingolimod as a mixture of diastereomers, as well as in a form of any of the single diastereomer or a pair thereof.

It should be further noted that the compound of formula (11) has two centers that must be hydrogenated for to obtain fingolimod - the five-membered ring and the nitro-group. Thus, in essence, the hydrogenation reaction may run via two possible intermediates (6) and (14), resp.:

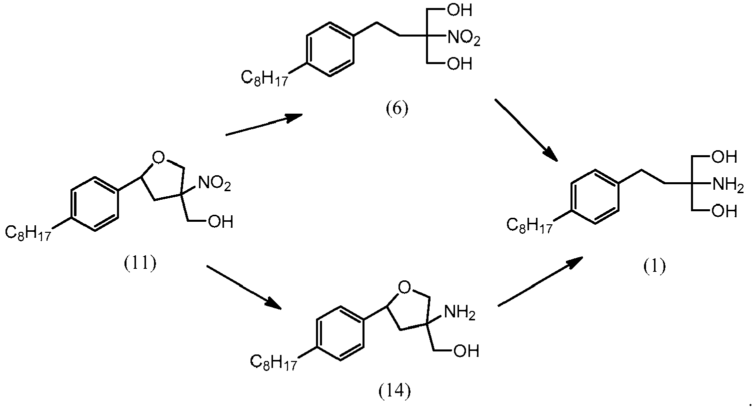

Both reactions may proceed in parallel or one of the reaction pathways may be significantly preferred. This depends primarily on the nature of the chosen catalyst, pH of the reaction mixture, nature of the solvent and the reaction temperature. For instance, the ring-opening reaction leading to (6) is preferred at higher temperatures, while hydrogenation reaction at lower temperatures runs preferably via the intermediate (14).

However, in general, it is not essential for purpose of the present invention whether the reaction will run via the compound (6) or compound (14).

If it is necessary or desirable, any of the intermediates (6) and (14) may be isolated for the reaction mixture, as a free compound or as an acid addition salt thereof, and subjected to the conversion to fingolimod of formula (1) in a separate step.

Similarly as the (11), the compound (14) exhibits two chiral carbons and may exist as any of four possible diastereomers.

### Variant b]

The present invention also refers to a convenient variant of the above processes, which comprises direct making the fingolimod of formula (1) by a catalytic hydrogenation of the compound (8) in the presence of a strong acid, e.g. p-toluenesulfonic acid or anhydrous hydrogen chloride. It is presumed that the reactive intermediate (15) formed by the action of the strong acid is preferentially transformed by the reaction with hydrogen directly to the compound (6), essentially without forming the compound (11). The conversion of (8) to (6) is then essentially a one-step technological process; however, contrary to a similar process disclosed in CN '869, the presence of strong anhydrous acid substantially increases the speed of the reaction and, accordingly, dramatically shortens the necessary reaction time. For instance, hydrogenation over palladium catalyst and in presence of anhydrous hydrogen chloride may proceed at a temperature, which is ambient or close to ambient, and at low pressure of hydrogen (about 1 bar and/or, in some embodiments, even less than 1 bar), whereby full conversion (96-98%) may be obtained in few hours.

The reaction partners (solvent, catalyst, strong acid) of this variant of the process are, mutatis mutandis, essentially the same as those disclosed above for the respective steps of variant a]. In an important aspect, the process generally runs at milder conditions than that in the variant a] (i.e. lower amount of the acid, lower pressure and lower temperature of the hydrogenation , typically from 10 to 60C, are necessary for the full conversion).

The compound (6) may be isolated from the reaction mixture, if desirable, by conventional procedures and may be subjected by a separate reduction reaction of the nitro-group. In such case, any suitable reductant may be used; except of a hydrogenation reaction with hydrogen under a presence of a hydrogenation catalyst, also a chemical reduction may be used, e.g. by a hydride, a dithionite, a borane, sodium sulfite etc.

However, in an advantageous mode, the compound (6) is not isolated from the reaction mixture and the hydrogenation of the compound (8) is directed in such a way that full conversion of the compound (8) to fingolimod of formula (1) is obtained. Typically, a full hydrogenation over palladium catalyst may be obtained at temperatures between 30 - 50°C and at a hydrogen pressure of between 1-50 bar.

The fingolimod compound obtained by the process of the present invention may be isolated as a free base or preferably in a form of an acid addition salt, advantageously in a form of hydrochloride, and purified by processes known in the art. For instance, fingolimod free base may be purified by a recrystallization from ethyl acetate, in which the crystalline polymorphic form A is obtained.

Fingolimod may be used as a pharmaceutically active compound for making pharmaceutical compositions for treatment various diseases, as shown in the art.

The present invention is illustrated by following non-limiting examples.

### EXAMPLES

### Example 1

### 3-nitro-1-(4-octylphenyl)propan-1-ol

A solution of 11.0 g of 3-nitro-1-(4-octylphenyl)propan-1-one(37.8 mmol) in 48 g THF, 3.6 g water and 1.2 g methanol was cooled to 0°C. Into the solution, 0.6 g of NaBH₄ (15.9 mmol) was added and the solution was stirred at the same temperature. After 2 hours, the reaction mixture was quenched by 65 g water. Then 80 g EtOAc was added and the aqueous phase was extracted with 2 × 20 g EtOAc. The combined organic phases were washed with 2 × 15 g water. Carbofiltration of the yellow organic phase with 0.4 g activated carbon followed by removal of the solvent by rotary evaporation provided 9.72 g of the title compound (33.1 mmol, 88%) as a colourless oily residue. Content of the impurity (13): 1.34% (HPLC).

### Example 2

### 3-nitro-1-(4-octylphenyl)propan-1-ol

The 3-nitro-1-(4-octylphenyl)propan-1-one (5 g, 17.16 mmol) was dissolved in dry THF(75 mL) under a nitrogen atmosphere. The solution was cooled to 0°C and 4M lithium borohydride solution in THF (2.145 mL, 8.58 mmol) was added over a period of 12 min. Stirring was continued at 0°C and followed by monitoring by HPLC. After 25 min HPLC showed that no starting material was present anymore. The reaction mixture was poured in ice-water and 4M aqueous hydrochloric acid was added (evolution of gas!) until pH<6. The mixture was extracted with diethyl ether (3 x 100 ml), washed with brine (100 ml), dried (sodium sulfate), filtered and evaporated to dryness to afford 3-nitro-1-(4-octylphenyl)propan-1-ol as a yellow oil in a yield of 4.72 g (94%).

### Example 3

### 3-(hydroxymethyl)-3-nitro-1-(4-octylphenyl)butane-1,4-diol

The 3-nitro-1-(4-octylphenyl)propan-1-ol (3.27 g, 11.15 mmol) was dissolved in methanol (11.15 ml) to give a turbid orange solution. To the resulting solution, triethyl amine (1.553 ml, 11.15 mmol) was added followed by formalin (37% solution in water, stabilized with 12% methanol) (5.02 ml, 66.9 mmol). The mixture was heated to 40°C and the reaction was monitored by HPLC. The HPLC after 120 minutes showed complete conversion of the starting material. Water (100 mL) was added giving a white emulsion. 1M aqueous hydrochloric acid was added until pH <6. The mixture was extracted with ethyl acetate (2x100 ml), washed with brine (25 ml), dried (sodium sulfate), filtered and evaporated to dryness to afford product 2 as a brown solid/oil in a yield of 3.54 g (97%).

The crude product was recrystallized from a mixture of heptane and ethyl acetate, yielding a white solid in a yield of 2.06 g (52%) with a purity of>99%.

### Example 4

### Preparation of compound (11)

3-(hydroxymethyl)-3-nitro-1-(4-octylphenyl)butane-1,4-diol (5 g, 14.10 mmol) was dissolved in 50 ml of toluene at 50°C. To this solution, 4-methylbenzenesulfonic acid hydrate (0.537 g, 2.82 mmol) was added and internal temperature was increased to reflux. Water was removed from reaction mixture by Dean-Stark distillation head. After 30 minutes, total conversion of the starting material was observed. Temperature of the reaction mixture was decreased to 21°C and organic layer was extracted four times with 20 ml of 0.5 mol aqueous solution of Na₂CO₃. Finally the organic layer was washed with 3x20 ml of water. Organic layer was dried with solid Na₂SO₄ and filtered with charcoal over celite. The solvent was removed by evaporator.

Yield: 4.2 g (89.3%) of the compound (11) [mixture of diastereomers].

Crude product can be crystallized from pentane with yield 80% and purity 99.9% (HPLC IN).

### Example 5

### Fingolimod

Autoclave vessel was loaded with p-toluenesulfonic acid monohydrate (8.04 g, 42.3 mmol), and with 3-(hydroxymethyl)-3-nitro-1-(4-octylphenyl)butane-1,4-diol (5 g, 14.13 mmol) dissolved in MeOH (100 ml) followed by the addition of palladium/C (1.5 g, 1.410 mmol). Autoclave was flushed twice with nitrogen and pressurized with hydrogen to 50 bar. The reaction mixture was hydrogenated at 50°C and 600rpm.Total reaction time was 230 min.

After this time, hydrogen pressure was interrupted and internal temperature decreased to 30°C. Reaction mixture was filtered over celite and methanol was evaporated (50°C, 200 mbar). Ethyl acetate (80 ml) was added to the white solid residue and the mixture was heated to 65°C. Then 40 ml of aqueous solution of Na2CO3 (0.5 mol) was added and ,at 50-55°C, the biphasic mixture was allowed to settle after vigorous stirring .The organic phase was separated and the aqueous phase was extracted with 80 ml ethyl acetate under the same conditions twice more.. The combined organic layers were washed with 4x30 ml of water.

Organic layer was dried with solid sodium sulphate at 55-60°C and partially evaporated to a mass of 30 g (Formation of off white crystals observed during evaporation).

The mixture was stored to freezer at -12°C for 18 h. The solid material was filtered off, washed with 2x5 ml of ethyl acetate and dried at 30°C, and 10 mbar for 120 min.

Yield: 3.3 g (76%) of off white solid material with purity 99.8% (HPLC IN).

### Example 6

### Fingolimod hydrochloride

2-amino-2-(2-(4-octylphenyl)ethyl)propane-1,3-diol (1 g, 3.24 mmol) was stirred with 2-propanol (5 ml). Into heterogenic mixture, anhydrous HCl in 2-propanol (3.3 ml, 21 %) was dropwise added. The mixture was warmed to 65°C. Solid material was completely dissolved. The solution was dropwise added into n-heptane (20 ml) for 5 min and intensively stirred. Formation of an off white precipitate was observed. Mixture was stirred for 60 min at 0°C. The solid material was filtered off, washed with 2x5 ml of n-heptane and dried at 35°C (10 mbar) for 120 min.

Yield: 0.98 g (88%) of off white solid material with purity 99.97% (HPLC IN).

### Example 7

### Fingolimod via Compound (6)

Autoclave vessel was loaded with 3-(hydroxymethyl)-3-nitro-1-(4-octylphenyl)butane-1,4-diol (10 g, 28.2 mmol) dissolved in MeOH (100 ml) followed by the addition of palladium /C (10%) (3.00 g, 2.82 mmol) and hydrogen chloride solution in 2-propanol (0.539 ml, 2.82 mmol). System was stirred under hydrogen pressure (less than 1 bar) at room temperature.

After 90 minutes of stirring, the pressure of hydrogen was increased to 35 bar and internal temperature increased from 21°C to 50°C (600 rpm). After 70 reaction minutes was reaction mixture filtered over celite and MeOH evaporated (40°C, 210-15 mbar) to dryness.

Yield: 8.3 g (93%) of pale grey solid material.

### Example 8

### Fingolimod

Autoclave vessel was loaded with (3-nitro-5-(4-octylphenyl)tetrahydrofuran-3-yl)-methanol (compound (11), 5 g, 14.91 mmol) dissolved in methanol (100 ml) followed addition of palladium/C (10%) (anhydrous) (1.586 g, 1.491 mmol) and hydrogen chloride solution in 2-propanol (11.23 ml, 59.6 mmol). The autoclave was flushed twice with nitrogen and pressurized with hydrogen up to 35 bars and heated to 100°C. The reaction mixture was stirred (600 rpm) at this temperature for 2 hours. After this time was internal temperature of reaction mixture decreased to 30°C and filtered over celite. MeOH was evaporated (40°C, 210-15 mbar) to dryness.

Yield: 4.2 g (86%) of grey solid material.

### Example 9

### Compound (14)

Autoclave vessel was loaded with (3-nitro-5-(4-octylphenyl)tetrahydrofuran-3-yl)methanol (2 g, 5.96 mmol) dissolved in toluene (60 ml) followed by the addition of palladium/C (5%) (0.063 g, 0.596 mmol). Autoclave was flushed twice with nitrogen and pressurized with hydrogen up to 35 bar at 30°C (600 rpm).

After 120 reaction minutes was reaction mixture filtered over celite and solvent evaporated (50°C, 90-15 mbar).

Yield: 1.8 g (90%) of dark grey solid material which contains mixture of desired product (14) and fingolimod in mutual ratio 70 / 30 (measured by HPLC IN).

### Example 10

### Purification of fingolimod base (procedure of example 28 of EP0627406 B1)

0.5 g of fingolimod was dissolved in 6 ml of ethyl acetate at reflux. The solution was allowed to cool to room temperature. As a result, a solid was formed rapidly. The solid was isolated by filtration over a P3-glass filter (reduced pressure) and air-dried overweekend at room temperature. White to off-white aggregates of thin flakes were obtained. The yield was 0.45 g.

| | |
|---|---|
| DSC: | Melting around 124-125 °C |
| XRPD: | See Figure 1. |

### Measurement conditions:

| | |
|---|---|
| Apparatus XRPD: detector | Bruker-AXS D8 vario, Θ/2Θ geometry, reflection mode, Vantec PSD |
| Start angle (2theta): | 2.0 ° |
| End angle (2theta): | 35.0 ° |
| Scan step width: | 0.02 ° |
| Scan step time: | between 0.2-2.0 seconds |
| Radiation type: | Cu |
| Radiation wavelength: | 1.54060 Å (K(alpha1)), primary monochromator used |

The invention having been described, it will be readily apparent to those skilled in the art that further changes and modifications in actual implementation of the concepts and embodiments described herein can easily be made or may be learned by practice of the invention, without departing from the scope of the invention as defined by the following claims.

## Claims

1. A process of making fingolimod of formula (1), 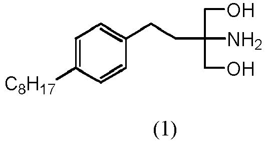 or an acid addition salt thereof,
comprising a step of reacting the compound of formula (11) 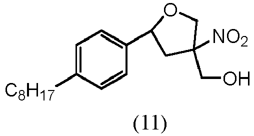 and/or a compound of formula (14) 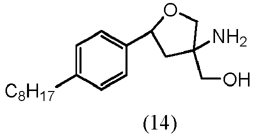 or an acid addition salt thereof,
in a solvent with hydrogen in the presence of a hydrogenation catalyst, preferably a palladium catalyst, and optionally converting fingolimod of formula (1) into an acid addition salt.

2. The process according to claim 1, wherein the solvent is an aliphatic or aromatic hydrocarbon, preferably toluene or an aliphatic alcohol, preferably methanol.

3. The process according to claims 1-2, wherein the reaction temperature is from 25 to 100°C.

4. A process according to claims 1-3, further comprising a step for making the compound of formula (11) comprising reacting the compound of formula (8) 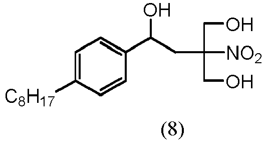 in a nonaqueous solvent with a strong acid, most preferably p-toluene sulfonic acid.

5. A process according to claim 4, wherein the compound (8) reacts with the strong acid in an aliphatic or aromatic hydrocarbon and/or in an aliphatic alcohol solvent, preferably at reflux.

6. A process according to claims 1-5 wherein both steps run sequentially without isolation of the compound (11) from the reaction mixture.

7. A process according to claims 1-6 further comprising a step of making the compound (8) comprising the reaction of compound (9) 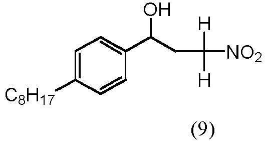 with a solution of formaldehyde in a mixture of water and methanol.

8. A process according to claims 1-7 further comprising a step of making the compound (9) by reacting the compound of formula (12) 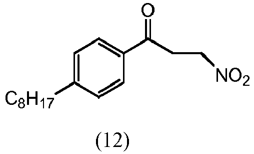 with lithium borohydride.

9. The compound of formula (11), 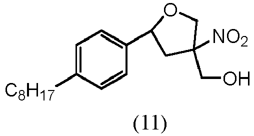 and/or a diastereomer thereof.

10. A process for making the compound of formula (11) comprising reacting the compound of formula (8) 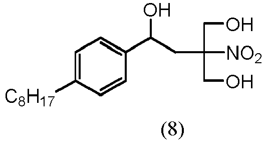 with a strong acid, most preferably p-toluene sulfonic acid.

11. A process according to claim 10, wherein the compound (8) reacts with the strong acid in a nonaqueous solvent, typically in an aliphatic or aromatic hydrocarbon and/or in an aliphatic alcohol solvent, preferably at reflux.

12. The process according to claims 10-11 comprising also isolating the compound (11) from the reaction mixture.

13. The process according to claims 10-12 comprising also a step of converting the compound (11) to fingolimod of formula (1).

14. The compound of formula (14) 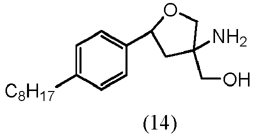 and/or an acid addition salt thereof.

15. A process of making fingolimod of formula (1), 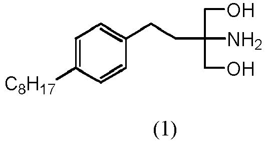 or an acid addition salt thereof,
comprising a step of reacting the compound of formula (8) 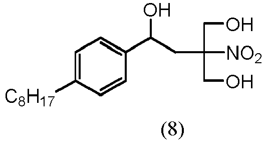 in a non-aqueous solvent with hydrogen in the presence of a hydrogenation catalyst, preferably a palladium catalyst, and in the presence of a strong acid, preferably p-toluene sulfonic acid or hydrogen chloride,
and optionally converting fingolimod of formula (1) into an acid addition salt.

16. The process according to claim 15, wherein the solvent is an aliphatic or aromatic hydrocarbon , preferably toluene, or an aliphatic alcohol, preferably methanol.

17. The process according to claims 15-16, wherein the intermediate compound of formula (6) 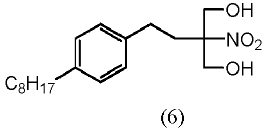 is isolated from the reaction mixture.

18. The process according to claim 17 further comprising a step of a conversion of the compound of formula (6) to fingolimod by reduction.

19. The process according to claims 15-16 wherein the intermediate compound of formula (6) is not isolated from the reaction mixture.

20. Use of compounds of formulas (11) and/or (14) for making fingolimod of formula (1), acid addition salts thereof and/or pharmaceutical compositions comprising the same.

## Patentansprüche

1. Verfahren zum Herstellen von Fingolimod der Formel (1), 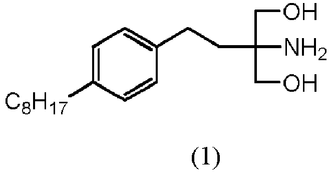 oder einem Säureadditionssalz davon,
das einen Schritt des Umsetzens der Verbindung der Formel (11) 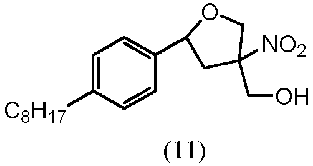 und/oder einer Verbindung der Formel (14) 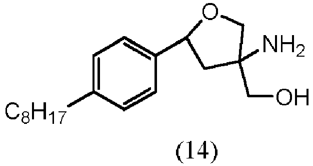 oder eines Säureadditionssalzes davon,
in einem Lösungsmittel mit Wasserstoff in der Gegenwart eines Hydrierkatalysators, bevorzugt eines Palladiumkatalysators, umfasst, und gegebenenfalls Umwandeln von Fingolimod der Formel (1) in ein Säureadditionssalz.

2. Verfahren nach Anspruch 1, wobei das Lösungsmittel ein aliphatischer oder aromatischer Kohlenwasserstoff, bevorzugt Toluol oder ein aliphatischer Alkohol, bevorzugt Methanol, ist.

3. Verfahren nach Ansprüchen 1-2, wobei die Reaktionstemperatur von 25 bis 100°C beträgt.

4. Verfahren nach Ansprüchen 1-3, das weiterhin einen Schritt zum Herstellen der Verbindung der Formel (11) umfasst, der Umsetzen der Verbindung der Formel (8) 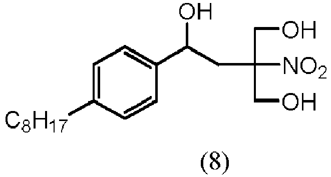 in einem nicht wässrigen Lösungsmittel mit einer starken Säure, am stärksten bevorzugt p-Toluolsulfonsäure, umfasst.

5. Verfahren nach Anspruch 4, wobei die Verbindung (8) mit der starken Säure in einem aliphatischen oder aromatischen Kohlenwasserstoff und/oder in einem aliphatischen alkoholischen Lösungsmittel reagiert, bevorzugt unter Rückfluss.

6. Verfahren nach Ansprüchen 1-5, wobei beide Schritte der Reihe nach ohne Isolierung der Verbindung (11) aus dem Reaktionsgemisch ablaufen.

7. Verfahren nach Ansprüchen 1-6, das weiterhin einen Schritt des Herstellens der Verbindung (8) umfasst, der die Reaktion der Verbindung (9) 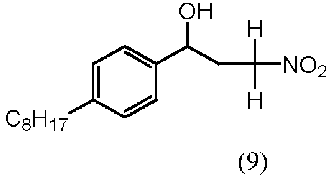 mit einer Lösung von Formaldehyd in einem Gemisch aus Wasser und Methanol umfasst.

8. Verfahren nach Ansprüchen 1-7, das weiterhin einen Schritt des Herstellens der Verbindung (9) durch Umsetzen der Verbindung der Formel (12) 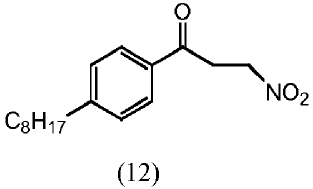 mit Lithiumborhydrid umfasst.

9. Verbindung der Formel (11), 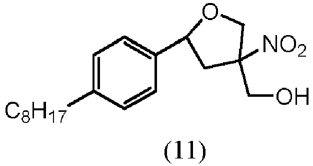 und/oder ein Diastereomer davon.

10. Verfahren zum Herstellen der Verbindung der Formel (11), das Umsetzen der Verbindung der Formel (8) 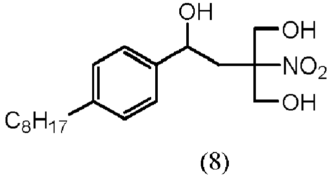 mit einer starken Säure umfasst, am stärksten bevorzugt p-Toluolsulfonsäure.

11. Verfahren nach Anspruch 10, wobei die Verbindung (8) mit der starken Säure in einem nicht wässrigen Lösungsmittel, typischerweise in einem aliphatischen oder aromatischen Kohlenwasserstoff und/oder in einem aliphatischen alkoholischen Lösungsmittel, bevorzugt unter Rückfluss reagiert.

12. Verfahren nach Ansprüchen 10-11, das auch Isolieren der Verbindung (11) aus dem Reaktionsgemisch umfasst.

13. Verfahren nach Ansprüchen 10-12, das auch einen Schritt des Umwandelns der Verbindung (11) zu Fingolimod der Formel (1) umfasst.

14. Verbindung der Formel (14) 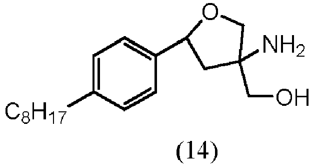 und/oder ein Säureadditionssalz davon.

15. Verfahren zum Herstellen von Fingolimod der Formel (1) 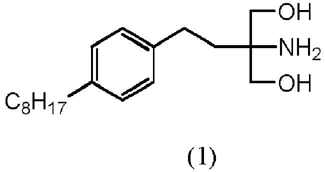 oder einem Säureadditionssalz davon,
das einen Schritt des Umsetzens der Verbindung der Formel (8) 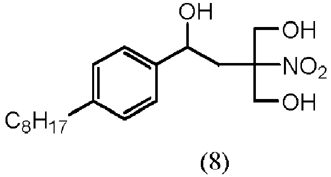 in einem nicht wässrigen Lösungsmittel mit Wasserstoff in der Gegenwart eines Hydrierkatalysators, bevorzugt eines Palladiumkatalysators, und in der Gegenwart einer starken Säure, bevorzugt p-Toluolsulfonsäure oder Chlorwasserstoff, umfasst,
und gegebenenfalls Umwandeln von Fingolimod der Formel (1) in ein Säureadditionssalz.

16. Verfahren nach Anspruch 15, wobei das Lösungsmittel ein aliphatischer oder aromatischer Kohlenwasserstoff, bevorzugt Toluol, oder ein aliphatischer Alkohol, bevorzugt Methanol ist.

17. Verfahren nach Ansprüchen 15-16, wobei die Intermediatverbindung der Formel (6) 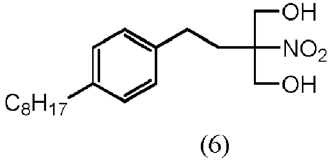 aus dem Reaktionsgemisch isoliert wird.

18. Verfahren nach Anspruch 17, das weiterhin einen Schritt einer Umwandlung der Verbindung der Formel (6) zu Fingolimod durch Reduktion umfasst.

19. Verfahren nach Ansprüchen 15-16, wobei die Intermediatverbindung der Formel (6) nicht aus dem Reaktionsgemisch isoliert wird.

20. Verwendung von Verbindungen der Formeln (11) und/oder (14) zum Herstellen von Fingolimod der Formel (1), Säureadditionssalzen davon und/oder pharmazeutischen Zusammensetzungen, die dieses enthalten.

## Revendications

1. Un procédé de fabrication de fingolimod de formule (1), 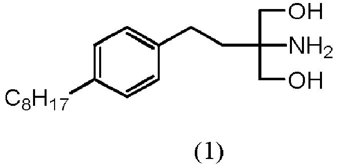 ou d'un sel d'addition d'acide en dérivant,
comprenant une étape de réaction du composé de formule (11) 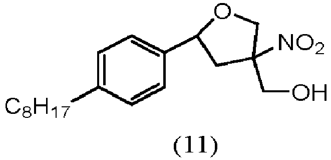 et/ou d'un composé de formule (14) 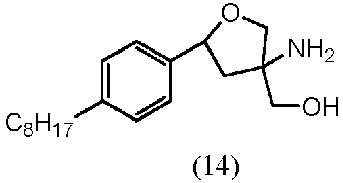 ou d'un sel d'addition d'acide en dérivant,
dans un solvant avec de l'hydrogène en présence d'un catalyseur d'hydrogénation, de préférence un catalyseur palladium, et éventuellement la conversion du fingolimod de formule (1) en un sel d'addition d'acide.

2. Le procédé selon la revendication 1, dans lequel le solvant est un hydrocarbure aliphatique ou aromatique, de préférence du toluène ou un alcool aliphatique, de préférence du méthanol.

3. Le procédé selon les revendications 1-2, dans lequel la température de réaction est de 25 à 100°C.

4. Un procédé selon les revendications 1-3, comprenant en outre une étape pour la fabrication du composé de formule (11) comprenant la réaction du composé de formule (8) 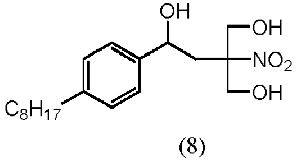 dans un solvant non aqueux avec un acide fort, plus préférablement de l'acide p-toluène sulfonique.

5. Un procédé selon la revendication 4, dans lequel le composé (8) réagit avec l'acide fort dans un hydrocarbure aliphatique ou aromatique et/ou dans un solvant d'alcool aliphatique, de préférence au reflux.

6. Un procédé selon les revendications 1-5 dans lequel les deux étapes sont mises en oeuvre séquentiellement, sans isolation du composé (11) du mélange réactionnel.

7. Un procédé selon les revendications 1-6, comprenant en outre une étape de fabrication du composé (8) comprenant la réaction du composé (9) 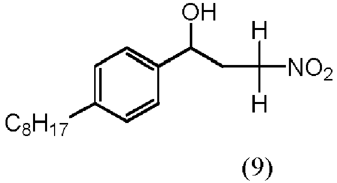 avec une solution de formaldéhyde dans un mélange d'eau et de méthanol.

8. Un procédé selon les revendications 1-7 comprenant en outre une étape de fabrication du composé (9) par réaction du composé de formule (12) 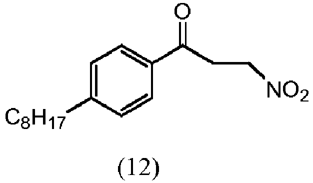 avec du borohydrure de lithium

9. Le composé de formule (11), 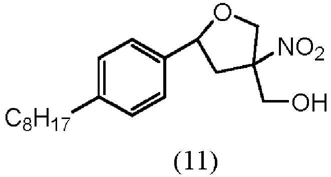 et/ou un diastéréomère en dérivant.

10. Un procédé de fabrication du composé de formule (11), comprenant la réaction du composé de formule (8) 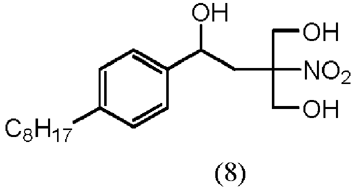 avec un acide fort, le plus préférablement de l'acide p-toluène sulfonique.

11. Un procédé selon la revendication 10, dans lequel le composé (8) réagit avec l'acide fort dans un solvant non aqueux, typiquement dans un hydrocarbure aliphatique ou aromatique et/ou dans un solvant d'alcool aliphatique, de préférence au reflux.

12. Le procédé selon les revendications 10-11, comprenant également l'isolation du composé (11) du mélange réactionnel.

13. Le procédé selon les revendications 10-12 comprenant également une étape de conversion du composé (11) en fingolimod de formule (1).

14. Le composé de formule (14) 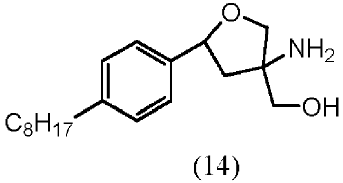 et/ou un sel d'addition d'acide en dérivant.

15. Un procédé de fabrication de fingolimod de formule (1), 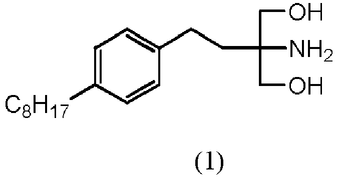 ou un sel d'addition d'acide en dérivant comprenant une étape de réaction du composé de formule (8) 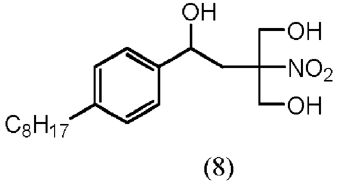 dans un solvent non-aqueux avec de l'hydrogène en présence d'un catalyseur d'hydrogénation, de préférence un catalyseur palladium, et en présence d'un acide fort, de préférence de l'acide p-toluène sulfonique ou du chlorure d'hydrogène,
et éventuellement la conversion du fingolimod de formule (1) en un sel d'addition d'acide.

16. Le procédé selon la revendication 15, dans lequel le solvant est un hydrocarbure aliphatique ou aromatique, de préférence le toluène, ou un alcool aliphatique, de préférence le méthanol.

17. Le procédé selon les revendications 15-16, dans lequel le composé intermédiaire de formule (6) 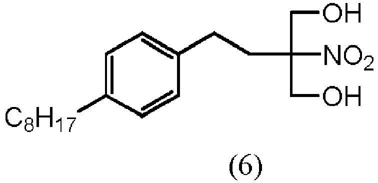 est isolé du mélange réactionnel.

18. Le procédé selon la revendication 17 comprenant en outre une étape de conversion du composé de formule (6) en fingolimod par réduction.

19. Le procédé selon les revendications 15-16 dans lequel le composé intermédiaire de formule (6) n'est pas isolé du mélange réactionnel.

20. Utilisation des composés de formules (11) et/ou (14) pour fabriquer la fingolimod de formule (1), des sels d'addition d'acide en dérivant et/ou des compositions pharmaceutiques les comprenant.
